Europäisches Patentamt

European Patent Office

Office européen des brevets

(19)

(11) Numéro de publication : **0 397 292 B1**

(12) # FASCICULE DE BREVET EUROPEEN

(45) Date de publication du fascicule du brevet :
**21.04.93 Bulletin 93/16**

(51) Int. Cl.⁵ : **C03C 17/23**

(21) Numéro de dépôt : **90201811.8**

(22) Date de dépôt : **23.12.85**

(54) **Procédé pour la formation d'une couche mince d'oxydes métalliques sur un substrat, notamment en verre, et son utilisation comme vitrage.**

(30) Priorité : **22.01.85 FR 8500854**
**03.07.85 FR 8510145**

(43) Date de publication de la demande :
**14.11.90 Bulletin 90/46**

(60) Numéro de publication de la demande initiale
en application de l'article 76 CBE : **0 192 009**

(45) Mention de la délivrance du brevet :
**21.04.93 Bulletin 93/16**

(84) Etats contractants désignés :
**AT BE CH DE FR GB IT LI LU NL SE**

(56) Documents cités :
**EP-A- 0 108 616**
**FR-A- 2 283 098**
**INSTRUMENTS AND EXPERIMENTAL TECH-**
**NIQUES, vol. 21, no. 6, partie 2, novembre/de-**
**cembre 1978, juin 1979, pages 1653-1655,**
**Plenum Publishing Corp. Consultants Bureau,**
**New York, US; B.P. KRYZHANOVSKII et al.:**
**"Production of transparent conducting coa-**
**tings of indium trioxide"**

(73) Titulaire : **SAINT-GOBAIN VITRAGE**
**INTERNATIONAL**
**"Les Miroirs" 18, avenue d'Alsace**
**F-92400 Courbevoie (FR)**

(72) Inventeur : **Sauvinet, Vincent**
**42 Rue des Dames**
**F-75017 Paris (FR)**
Inventeur : **Trouve, Maurice**
**8 Rue Gauthier de Nemours**
**F-77140 Nemours (FR)**
Inventeur : **Bletry, Jean**
**641 Route de la Pageonnière**
**F-38330 Saint-Ismier (FR)**
Inventeur : **Bonnaud, Micheline**
**2Bis Rue Louis Blanc**
**F-92240 Malakoff (FR)**

(74) Mandataire : **Luziau, Nelly et al**
**Saint Gobain Recherche 39, Quai Lucien**
**Lefranc BP 135**
**F-93303 Aubervilliers Cédex (FR)**

## Description

La présente demande est une demande divisionnaire de la demande de brevet européen publiée sous le numéro 198 009, appelée ci-après demande principale.

La présente invention concerne la formation de couches minces d'oxydes métalliques sur un substrat, notamment en verre, par pyrolyse de poudres de composés métalliques projetés sur ledit substrat, ces couches minces colorées ou non ayant essentiellement des propriétés de basse émissivité, de conduction électrique et de transparence.

Pour qu'un composé métallique pulvérulent puisse former un revêtement de bonne qualité sur un substrat, il faut qu'il puisse être distribué régulièrement, qu'il se décompose avec un rendement suffisant et ce, à une température ne dépassant pas 650°C ou 700°C si le substrat à revêtir est en verre et qu'il contienne une proportion de métal suffisante pour conduire à une couche d'oxyde d'épaisseur notable en un court instant (en particulier si ce substrat se déplace rapidement par rapport aux moyens distributeurs de la poudre, comme c'est le cas pour un ruban de verre à la sortie d'un bain dit "float").

On sait déjà réaliser sur des substrats en verre un revêtement d'oxyde d'étain que l'on forme en distribuant sur le substrat, de l'oxyde de dibutyl étain en poudre (DBTO) (voir par exemple les publications françaises de brevets FR 2 380 997 et 2 391 966), ou du difluorure de dibutyl étain en poudre (DBTF) (voir par exemple le brevet européen 0 039 256 et la publication française de brevet n° 2 542 636).

Ces poudres conduisent certes à des couches satisfaisantes ; cependant, elles sont colorées en réflexion pour les épaisseurs nécessaires à l'obtention de propriétés électroniques intéressantes. Or, la couleur peut ne pas plaire ou ne pas être adaptée au style de la structure environnante. Par ailleurs, de légères variations d'épaisseur de couches entraînent des irrégularités de couleur.

Pour obtenir une parfaite uniformité de la couleur, il faut respecter scrupuleusement certaines conditions, en particulier une qualité constante et bien déterminée de la poudre, et des réglages très précis des installations servant à la distribuer.

La couleur elle-même et les conditions opératoires qu'il est nécessaire de respecter pour qu'elle soit parfaitement uniforme constituent des inconvénients de ces couches à base d'oxyde d'étain.

On a aussi utilisé des composés d'indium, non pas en poudre, mais en solution, en particulier des acétylacétonates d'indium, pour fabriquer des couches minces sur du verre. Mais ces composés sous forme de solution, ne se pyrolysent qu'à des vitesses insuffisantes. Ils ne conviennent donc pas pour être appliqués sur un ruban de verre défilant à vitesse élevée à la sortie d'un "bain float".

La présente invention concerne donc la formation de couches minces transparentes et électroconductrices présentant les propriétés souhaitées sans avoir les inconvénients des couches à base d'oxyde d'étain, c'està-dire une coloration lorsqu'elles sont observées en réflexion.

En outre, ces couches doivent pouvoir être formées par pyrolyse de composés métalliques avec un rendement suffisamment élevé pour être compatible avec le défilement rapide du substrat.

Le procédé selon l'invention, pour former des couches minces transparentes et électroconductrices d'oxydes métalliques sur un substrat, notamment en verre, consiste à projeter sur le substrat porté à température élevée des composés métalliques en poudre en suspension dans un gaz vecteur qui, au contact du substrat chaud, se décomposent et s'oxydent pour former lesdits oxydes métalliques. Le procédé se caractérisé en ce qu'on projette sur le substrat de la poudre formée par du formiate d'indium ou par un mélange de formiate d'indium et d'un ou plusieurs composés métalliques ayant une température de décomposition du même ordre de grandeur que celle du formiate, la poudre se pyrolysant au contact du substrat en formant une couche à base d'oxyde d'indium.

Avantageusement, après dépôt et formation de la couche d'oxyde métallique sur le substrat, celle-ci subit un traitement thermique destiné à modifier ses propriétés électroniques et, en particulier, ses propriétés d'émissivité et de résistivité, en vue de les amener à la valeur désirée.

Dans un premier mode de réalisation, on fait séjourner le substrat revêtu de sa couche, dans une enceinte, chauffée à une température d'au moins 300°C et compatible avec le substrat, sous atmosphère contrôlée, pendant un temps qui est fonction de la température et de l'atmosphère, et qui peut s'échelonner de quelques secondes à plusieurs heures.

Dans un second mode de réalisation, on soumet le substrat revêtu de la couche d'oxyde métallique à un chauffage intense, mais pendant un temps inférieur à la seconde, en atmosphère contrôlée.

Selon ce second mode de réalisation, on peut obtenir le chauffage intense grâce à une flamme, en particulier celle d'un brûleur placé en regard et relativement au substrat revêtu.

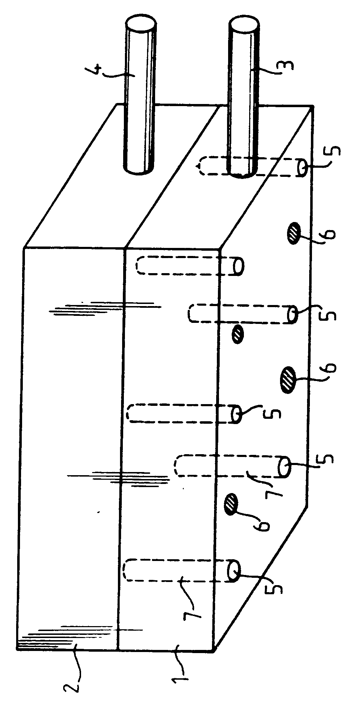

L'invention sera maintenant décrite plus en détail en référence aux exemples et à la figure jointe qui représente une vue schématique d'un brûleur pour mettre en oeuvre le traitement thermique selon le second mode de réalisation.

Le formiate d'indium peut être le seul composé métallique de la poudre, mais avantageusement il peut être associé à un ou plusieurs autres composés ayant des températures de décomposition du même ordre que celles du formiate d'indium.

Comme formiate d'indium en poudre, on peut avantageusement utilisé celle obtenue par le procédé décrit à la demande de brevet européen principal. Ce procédé consiste à attaquer l'indium par un acide, puis à précipiter l'hydroxyde d'indium en faisant réagir, sur le sel d'indium ainsi obtenu, de l'ammoniaque à une température voisine de l'ébullition, à laver et à sécher le précipité et à faire réagir ce dernier avec l'acide formique.

Ainsi, en utilisant HCl comme acide, le procédé de fabrication peut être résumé par les équations suivantes :

$$In + 3HCl \rightarrow InCl_3 + 3/2\ H_2$$
$$InCl_3 + 3NH_4OH \rightarrow In(OH)_3 + 3ClNH_4$$
$$In(OH)_3 + 3HCOOH \rightarrow In(HCOO)_3 + 3H_2O.$$

Au lieu de l'acide chlorhydrique, on peut aussi bien utiliser l'acide nitrique ou un autre acide.

Pour obtenir des couches très conductrices, on peut associer au formiate d'indium des composés d'étain en poudre et en particulier du DBTO et/ou du DBTF qui interviennent alors dans des proportions pondérales pouvant aller jusqu'à 30 %. Ces composés effectuent un dopage cationique de l'oxyde d'indium. En effet, le remplacement d'un atome d'indium par un atome d'étain introduit dans la couche un électron libre. Par ce dopage, on augmente donc la densité des porteurs de charge et la conductivité de la couche.

La poudre de formiate d'indium ou le mélange de poudres peut être projeté sur le substrat, notamment un ruban de verre, à l'aide d'une buse, par exemple par l'une des buses décrites dans les demandes de brevet français publiées sous les n° 2 542 636 et 2 542 637 précédée du dispositif de répartition décrit dans la demande de brevet français publiée sous le n° 2 548 556.

Le dopage peut également être effectué avec des composés gazeux, tels que $SnCl_4$ ou des organo-stanniques gazeux, tels que $BuSnCl_3$. Dans ce cas, le dopant est mélangé avec le gaz dans lequel la poudre est en suspension, et/ou avec les gaz qui servent à l'accélération et/ou à l'homogénéisation de la poudre. Le dopant peut encore être aspiré par la buse dans la chambre de contrôle décrite dans la demande de brevet français publiée sous le n° 2 542 636 et/ou encore être amené à la sortie de la buse de projection par une conduite spéciale.

Comme indiqué précédemment, après dépôt et formation de la couche d'oxyde métallique sur le substrat, celle-ci subit avantageusement un traitement thermique.

Par ce traitement, on cherche généralement à augmenter le nombre de lacunes d'oxygène de la couche de façon à abaisser l'émissivité de ladite couche, donc à améliorer ses propriétés de basse émissivité. Mais on peut aussi, au contraire, diminuer le nombre de lacunes d'oxygène de la couche de façon à augmenter l'émissivité de ladite couche, et donc à amoindrir ses propriétés de basse émissivité qu'elle possédait préalablement.

Suivant un premier mode de réalisation, ce traitement thermique est constitué par un séjour du substrat revêtu dans une enceinte chauffée, sous atmosphère contrôlée, séjour qui est plus ou moins long, qui dépend de la température, du caractère réducteur de l'atmosphère, et qui peut s'échelonner de quelques secondes à plusieurs heures.

L'amélioration des propriétés de basse émissivité demandera un traitement thermique en atmosphère réductrice ou éventuellement neutre.

Dans le cas d'une atmosphère réductrice, celle-ci peut être constituée par une atmosphère de composition identique à celle du "bain float", à savoir 90 % d'azote et 10 % d'hydrogène.

Par ce traitement en atmosphère réductrice ou neutre, on augmente la concentration en lacunes d'oxygène dans la couche, ce qui entraîne un dopage anionique favorable à l'augmentation de la conduction électrique et de la réflexion infrarouge de la couche. Selon la durée et la température du traitement, on peut faire varier considérablement la densité des porteurs de charges, par exemple de $1.10^{20}$ à $20.10^{20}$ porteurs par $cm^3$ et leur mobilité, par exemple de 10 à 50 $cm.V^{-1}.s^{-1}$.

Ce traitement peut être pratiqué immédiatement après la formation de la couche d'oxyde, directement sur la ligne de fabrication du verre, par exemple dans une étenderie de recuisson, de manière à profiter de la chaleur du verre.

Il peut être également pratiqué plus tard sur une installation séparée de la ligne de fabrication du verre (arche de recuisson annexe, ligne de trempe, de bombage).

Dans ce cas, on réchauffe le substrat revêtu jusqu'à une température d'au moins 300°C compatible avec le substrat, on le maintient en atmosphère réductrice ou neutre à cette température élevée pendant un temps d'autant plus court que la température est plus élevée et que l'atmosphère est plus réductrice (ce temps de maintien pouvant s'échelonner de quelques secondes à plusieurs heures), puis on le refroidit sous atmosphère réductrice ou neutre au moins jusqu'à une température où le contact avec une atmosphère oxydante n'altère

EP 0 397 292 B1

plus les performances de la couche.

Avantageusement, le refroidissement contrôlé sous atmosphère réductrice ou neutre est pratiqué jusqu'à une température qui est au maximum de l'ordre de 300°C. Dans le cas de traitement simultané à la trempe, les buses de soufflage du gaz de trempe sont alimentées par le gaz réducteur ou neutre nécessaire au traitement.

En résumé, l'efficacité du traitement de la couche augmente avec :

. le caractère réducteur de l'atmosphère de traitement, par exemple avec le pourcentage d'hydrogène d'un mélange $N_2$ + X % $H_2$ où X est positif ou nul, notamment X = 10 ou X = O,

. la température, celle-ci étant comprise entre 300 et 650°C,

. le temps, qui peut varier de quelques secondes à quelques heures.

Suivant un second mode de réalisation, ce traitement thermique est obtenu par un chauffage intense en atmosphère contrôlée, réductrice ou au contraire oxydante suivant la finalité recherchée, pendant un temps inférieur à la seconde. Matériellement cela est obtenu en soumettant le substrat revêtu de la couche d'oxyde métallique à l'action d'au moins une flamme, réductrice ou au contraire oxydante, suivant que l'on désire augmenter ou au contraire amoindrir les propriétés de basse émissivité de la couche ainsi traitée, réduire l'oxyde en métal ou au contraire oxyder le métal.

Ce traitement est alors avantageusement réalisé par défilement du substrat revêtu dans la flamme d'au moins un brûleur alimenté en gaz dont les caractéristiques réductrices ou oxydantes sont contrôlées et adaptées à la nature réductrice ou oxydante souhaitée dudit traitement.

Il s'agit donc d'un traitement immédiat (temps de traitement inférieur à la seconde), ne nécessitant qu'une installation réduite, simple, peu coûteuse et facile à conduire.

L'échauffement du substrat à la flamme peut être faible (inférieure à 70°C) dans la mesure où la flamme est disposée par rapport audit substrat du côté du revêtement, si bien que le niveau de trempe d'un substrat en verre préalablement trempé est conservé, ou que des substrats sensibles à la chaleur, en matériau autre que le verre ou comportant en association avec le verre d'autres matériaux tels que le polyvinylbutyral (PVB), ne sont pas altérés.

Le ou les brûleurs nécessaires au traitement de la couche sont alimentés en un mélange de gaz comburant (oxygène ou mélange de gaz neutre et d'oxygène) et de gaz combustible contenant de l'hydrogène et/ou du carbone (hydrogène, monoxyde de carbone, alcane, alcène ou alcyne gazeux).

Les proportions de comburant et de combustibles sont telles que le mélange n'est pas stoechiométrique, mais contient au contraire soit un excès de gaz réducteur, soit un excès de gaz oxydant, suivant que l'on désire effectuer un traitement réducteur ou un traitement oxydant.

Les brûleurs utilisables sont, de préférence pour des raisons de sécurité et de facilité d'emploi, des brûleurs linéaires à mélange externe.

Ces brûleurs sont du type illustré par la figure jointe, c'est-à-dire qu'ils possèdent deux chambres 1 et 2 d'amenée des gaz combustible et comburant, elles-mêmes alimentées par les tuyauteries 3 et 4. Chacune de ces chambres 1 et 2 débouche à l'extérieur du brûleur par des trous 5 et 6, les trous 5 délivrant le gaz de la chambre supérieure 2 étant reliés à cette chambre 2 par des conduites 7 qui traversent de façon étanche la chambre inférieure 1. Pour un bon mélange des gaz, les trous 5 et 6 sont disposés en quinconce. Dans la mesure où de grandes longueurs de brûleurs sont nécessaires, des déflecteurs non figurés, ou une forme en biseau des chambres 1 et 2, peuvent être prévus pour assurer une égalité de distribution des gaz d'un bout à l'autre du brûleur.

Des brûleurs de ce type sont distribués par la Société française "AIR LIQUIDE" sous la dénomination "brûleurs FMT".

La couche d'oxyde obtenue sur un substrat chauffé, par projection d'une poudre de formiate d'indium, associée ou non à des composés d'étain aptes à agir comme dopants vis-à-vis de l'indium, tels que l'oxyde de dibutyl étain (DBTO), et/ou le difluorure de dibutyl étain (DBTF), est par fabrication trop oxydée pour posséder de bonnes caractéristiques de basse émissivité et de basse résistivité. On peut lui faire subir le traitement de "brûlage" selon l'invention, de façon à améliorer ses caractéristiques. Le réglage des débits et des proportions de gaz oxydant et réducteur dépend de la nature des gaz, de l'installation et des conditions opératoires, et est fonction de l'émissivité et de la résistivité désirées pour la couche.

Il est possible de déterminer expérimentalement pour une installation donnée et pour des conditions opératoires données (vitesse de défilement du substrat, distance vis-à-vis des brûleurs) les réglages de débit de gaz de façon à établir les débits limites pour réduire totalement la couche à l'état métallique, l'apparition de cet état métallique se reconnaissant à ce que la couche change d'aspect, acquiert une certaine réflectivité dans le visible qui peut aller jusqu'à la réflectivité d'un miroir et peut, dans certains cas, en particulier pour l'oxyde d'indium transformé, devenir peu adhérente au substrat.

Pour cela, on règle les proportions respectives de gaz oxydant et de gaz réducteur de façon à disposer

4

d'un mélange légèrement plus réducteur que le mélange stoechiométrique, soit 10 % environ en plus de gaz réducteur par rapport au débit correspondant au mélange stoechiométrique. Ensuite, on pratique le traitement à la flamme de la couche sur divers échantillons en augmentant progressivement le débit global du mélange jusqu'à obtenir la réduction de la couche à l'état métallique. Ce réglage de débits limites étant obtenu, on sait que les réglages permettant d'atteindre les niveaux de basse émissivité et de basse résistivité désirés s'obtiendront en diminuant le débit de gaz réducteur par rapport au débit limite préalablement déterminé.

Grâce à cette technique d'échauffement rapide en atmosphère contrôlée, on a pu obtenir les propriétés optimales de basse émissivité et de basse résistivité d'une couche à base d'oxyde d'indium, déposée par pyrolyse de poudre comme indiqué précédemment, en faisant défiler le substrat revêtu de la couche, à 1 cm sous un brûleur linéaire à mélange externe, ayant, comme longueur, la largeur de la couche à traiter, à une vitesse de 3 cm/s, le débit d'oxygène étant de 1,9 l par minute et par centimètre de longueur de brûleur, le débit d'hydrogène étant de 4 l/min.cm.

En variant la vitesse de défilement d'un même substrat et en la portant à 5 cm/s, on a eu besoin d'un débit d'oxygène de 2,3 l/min.cm et un débit d'hydrogène de 4,7 l/min.cm.

En augmentant encore la vitesse de défilement de façon à ce qu'elle atteigne la vitesse de défilement du ruban de verre dans les installations "float" par exemple 20 cm/s, on a eu besoin pour obtenir les performances maximales de la couche, de l'ordre de 6 l/min. cm d'oxygène et 13 l/min.cm d'hydrogène.

Avec l'un ou l'autre de ces traitements thermiques, on a pu traiter des couches obtenues par pyrolyse d'une poudre de formiate d'indium et de 4 % en poids de DBTO et obtenir les résultats énumérés dans les exemples qui suivent :

## EXEMPLE I

|  | Avant traitement | Après traitement |
| --- | --- | --- |
| Epaisseur (Angströms) | 900 | 900 |
| Coef. moyen de réflexion IR | 0,33 | 0,70 |
| Emissivité | 0,67 | 0,30 |
| Facteur énergétique de transmission (%) | 74,5 | 76,0 |
| Facteur de transmission lumineuse (%) | 74,3 | 79,0 |
| Résistance d'un carré (ohms) | 205 | 35 |

Pour obtenir une telle couche, on a utilisé une poudre à base de formiate d'indium mélangée avec 4 % en poids de DBTO, qu'on a distribué sur un ruban de verre défilant à 18 m/min, dont la température de surface était de 600°C. Après pyrolyse de la poudre, on a soumis le substrat en verre ainsi revêtu à un traitement thermique consistant en une recuisson dans une enceinte à 600°C pendant 2 min sous atmosphère d'azote, puis en un refroidissement progressif jusqu'à 300°C, toujours sous atmosphère non oxydante, d'azote pendant 2 min.

**EXEMPLE II**

|  | Avant traitement | Après traitement |
|---|---|---|
| Epaisseur (Angströms) | 1900 | 1900 |
| Coef. moyen de réflexion IR | 0,53 | 0,87 |
| Emissivité | 0,47 | 0,13 |
| Facteur énergétique de transmission (%) | 77 | 72 |
| Facteur de transmission lumineuse (%) | 81,3 | 83,0 |
| Résistance d'un carré (ohms) | 92 | 11 |

**EXEMPLE III**

Dans les mêmes conditions que pour les exemples I et II, sauf en ce qui concerne le débit de poudre distribué sur le substrat en verre, on a réalisé une couche rouge mauve en réflexion, légèrement verte en transmission, dont les caractéristiques avant et après traitement thermique en enceinte sous atmosphère non oxydante sont les suivantes :

|  | Avant traitement | Après traitement |
|---|---|---|
| Epaisseur (Angströms) | 3000 | 3000 |
| Coef. moyen de réflexion IR | 0,76 | 0,89 |
| Emissivité | 0,24 | 0,11 |
| Facteur énergétique de transmission (%) | 78,8 | 66,0 |
| Facteur de transmission lumineuse (%) | 85,8 | 82 |
| Résistance d'un carré (ohms) | 25 | 7,5 |

**EXEMPLE IV**

On a soumis la même couche que celle de l'exemple II, puis de l'exemple III, avant traitement thermique, à la flamme d'un brûleur alimenté en un mélange d'oxygène et d'hydrogène comme décrit précédemment. Quelle que soit la vitesse de défilement sous la flamme du substrat revêtu, avec bien entendu des débits de gaz adaptés, on a obtenu après traitement, des couches ayant les mêmes caractéristiques que celles obtenues après traitement, respectivement dans les exemples II et III.

De la même façon que le premier type de traitement thermique, ce traitement par échauffement intense

6

mais court, par exemple dans la flamme d'un brûleur, peut être pratiqué immédiatement après la fabrication et le revêtement du verre, par exemple sur la ligne float même, mais il peut tout aussi bien être réalisé plus tard. C'est d'ailleurs ce qui est inévitable lorsque le verre doit être trempé ou feuilleté. Dans ce cas, le verre revêtu est découpé, trempé ou feuilleté, puis traité thermiquement.

Ainsi donc, avec l'un ou l'autre des deux traitements thermiques proposés, on a pu obtenir des couches à base de formiate d'indium et d'un ajout de DBTO ou de DBTF, ayant des résistivités électriques de l'ordre de 2 x $10^{-4}$ ohm.cm. On a pu également en modulant l'intensité du traitement thermique, obtenir des performances des couches, intermédiaires entre les performances de la couche non traitée et des performances optimales mesurées sur une couche ayant subi le traitement le plus intense.

Avec de tels traitements thermiques, on peut aussi, au lieu d'abaisser la résistivité et l'émissivité d'une couche, au contraire l'augmenter. Ainsi, à partir d'un substrat revêtu ayant une résistance carré de 15 ohms, on a pu atteindre une résistance de 16 ohms, ou de 2000 ohms en faisant défiler le substrat à 1 cm sous le brûleur, respectivement à 10 cm/s et à 6 cm/s, le débit d'oxygène dans les deux cas étant de 2,4 l/min.cm et celui d'hydrogène étant de 3 l/min.cm. Un tel traitement, conduisant à l'abaissement de la résistivité et de l'émissivité, est bien entendu également possible avec le premier mode de traitement thermique : il suffit de prévoir une atmosphère contrôlée non plus réductrice, mais oxydante.

On peut également avec l'un ou l'autre des deux modes de traitement thermique proposés, créer sur un même vitrage revêtu des zones de conductions électriques différentes par application de traitements thermiques différents. Ce genre de traitements thermiques différenciés suivant les zones d'un même vitrage est particulièrement aisé quand on met en oeuvre le second type de traitement thermique. Pour cela, on modifie localement ou momentanément les conditions d'exposition de la couche aux moyens de chauffage.

Dans cet ordre d'idée, on fait varier la vitesse de défilement du substrat revêtu à traiter par rapport au brûleur en fonction du résultat désiré. Avec un brûleur disposé transversalement par rapport à la direction de défilement du substrat, en modulant la vitesse de défilement dudit substrat, on a obtenu des couches à bandes transversales ayant des propriétés différenciées.

On a pu obtenir aussi des bandes longitudinales dans le sens de déplacement du substrat, à caractéristiques électriques et optiques différentes en ajoutant, face à ces zones, des brûleurs supplémentaires ou en effectuant le traitement sur toute la largeur du substrat à l'aide d'une pluralité de brûleurs réglés différemment les uns des autres.

Ces traitements thermiques et, en particulier, celui par brûleurs, sont donc particulièrement adaptés à la fabrication de vitrages à couches ayant des zones à propriétés électriques, thermiques, optiques, différenciées. Ainsi, par exemple, des vitrages chauffants à couches minces possédant des résistances plus ou moins importantes suivant les zones pourront être fabriqués.

Le second mode de traitement thermique proposé autorise, comme déjà dit, la fabrication de vitrages trempés ou feuilletés à couches minces.

En effet, alors que le premier mode de traitement thermique proposé altère la trempe, empêche le traitement de feuilletés ou de substrats non résistants à la température, le second mode de traitement thermique proposé est si bref que le substrat n'a pas le temps d'être échauffé, en particulier si le moyen de chauffage est disposé du côté de la couche. En conséquence, les intercalaires en P.V.B. (polyvinylbutyral) ou autres, des vitrages feuilletés, les substrats non résistants à la chaleur, les contraintes obtenues par la trempe, sont conservés sans altération.

Ce second mode de traitement thermique est donc particulièrement adapté à la fabrication de vitrages trempés à couches minces, le niveau de trempe étant élevé et autorisant en particulier l'usage de tels vitrages en tant que vitrages pour automobiles (c'est-à-dire avec une fragmentation telle que définie dans le règlement 43 des Nations Unies pour l'homologation des vitrages automobiles, le niveau d'émissivité et de résistivité étant également de bonne qualité, c'est-à-dire une émissivité inférieure à 0,15 et une résistivité inférieure à $3.10^{-4}$ ohm. cm.

Il est donc également particulièrement adapté à la fabrication de vitrages feuilletés comportant au moins une feuille de verre revêtue d'une couche mince nécessitant un traitement thermique pour acquérir ses caractéristiques définitives.

Ce second mode de traitement thermique a été décrit en utilisant un ou plusieurs brûleurs à gaz, remarquables pour leur simplicité, mais d'autres moyens de chauffage aptes à fournir en moins d'une seconde une énergie élevée peuvent aussi être utilisés ; ainsi, par exemple, une torche à plasma micro-onde alimentée en gaz réducteur ou au contraire oxydant suivant que l'on désire, comme déjà dit, soit réduire l'oxyde ou abaisser l'émissivité et la résistivité de la couche, soit au contraire oxyder le métal ou augmenter l'émissivité et la résistivité de la couche.

L'invention a été décrite en prenant des vitrages, destinés par exemple aux bâtiments (vitrages bas émissifs, vitrages chauffants, et en général vitrages utilisant les propriétés optiques et/ou électriques des couches), ou à l'automobile (vitrages chauffants, vitrages de sécurité utilisant les propriétés de conduction de la couche pour détecter une effraction, vitrages antenne...), mais elle peut aussi trouver une application intéressante dans le revêtement de substrats divers, isolants en particulier, articles en verre tels que bouteilles, flacons, verreries diverses, éléments d'optique, lampes d'éclairage, fibre de verre, ou encore articles en silice, matériaux réfractaires, alumine.

Le matériel utilisé pour projeter la poudre ou le mélange de poudres sur des objets séparés est alors généralement différent des buses auxquelles on s'est référé ci-dessus. On utilisera, par exemple, des pistolets classiques de pulvérisation de poudres.

La description ci-dessus a exclusivement concerné des composés en poudre, à base de formiate d'indium et leur emploi sous forme de poudre.

Cependant, ces poudres à base de formiate d'indium peuvent également être transformées et être employées en phase autre que solide et pulvérulente.

En particulier, la poudre peut être mise en solution, notamment dans du méthanol et le composé à base de formiate d'indium en solution, peut être distribué sur un substrat, y être pyrolysé et former ainsi une couche d'oxyde métallique qui pourra subir un traitement thermique apte à modifier les caractéristiques de la couche initiale.

Les traitements thermiques pratiqués postérieurement au dépôt de la couche ont été décrits comme étant appliqués à une couche d'oxyde d'indium dopée ou non à l'étain obtenue par pyrolyse d'une poudre à base de formiate d'indium ; mais ils peuvent également s'appliquer avec succès à toute couche à base d'oxyde d'indium, dopée ou non, à l'étain ou autre, obtenue par une autre voie, par exemple pyrolyse liquide, CVD, ou technique sous vide, que le composé de départ soit du formiate ou autre.

Ces traitements thermiques peuvent même s'appliquer à toute autre couche métallique qui, comme la couche à base d'oxyde d'indium, est non stoechiometrique, ainsi par exemple les couches à base d'oxyde de vanadium, de Zn, de Sn...

Ces traitements thermiques peuvent même permettre d'obtenir, à partir de couches d'oxyde métallique, des couches de métal. L'obtention d'une couche de métal à partir d'une couche d'oxyde a été décrite comme une phase du réglage des brûleurs servant aux traitements thermiques, mais l'obtention d'une telle couche de métal peut être le but final.

Les traitements thermiques peuvent également être mis en oeuvre pour recristalliser une couche mal cristallisée ou amorphe, en particulier pour modifier ses propriétés électroniques.

## Revendications

1. Procédé de formation d'une couche mince transparente et électroconductrice d'oxyde(s) métallique(s) sur un substrat, notamment du verre, consistant à projeter, sur ledit substrat porté à température élevée, des composés métalliques en poudre en suspension dans un gaz vecteur, qui, au contact du substrat chaud, se décomposent et s'oxydent pour former ladite couche d'oxyde(s) métallique(s), caractérisé en ce qu'on projette sur le substrat de la poudre formée par du formiate d'indium ou par un mélange de formiate d'indium et d'un ou plusieurs composés métalliques ayant une température de décomposition du même ordre de grandeur que celle du formiate d'indium, la poudre se pyrolysant au contact du substrat en formant une mince couche à base d'oxyde d'indium.

2. Procédé conforme à la revendication 1, caractérisé en ce qu'on projette, sur le substrat, une poudre en mélange comprenant du formiate d'indium et de l'oxyde de dibutyl étain et/ou du difluorure de dibutylétain.

3. Procédé conforme à la revendication 1, caractérisé en ce qu'on projette, sur le substrat, de la poudre de formiate d'indium et un composé gazeux de l'étain choisi dans le groupe formé par $SnCl_4$ et $BuSnCl_3$, mélangé au gaz vecteur dans lequel ladite poudre est en suspension.

4. Procédé conforme à l'une des revendications 1 à 3, caractérisé en ce que la couche mince obtenue est soumise à un traitement thermique, en atmosphère réductrice ou oxydante suivant que l'on veut réduire ou oxyder la couche, augmenter le nombre des lacunes d'oxygène pour abaisser l'émissivité et la résistivité de la couche et donc améliorer ses propriétés de basse émissivité et de résistivité, ou au contraire, diminuer le nombre de lacunes d'oxygène pour élever l'émissivité et la résistivité de la couche et donc amoindrir les propriétés de basse émissivité qu'elle possédait préalablement.

5. Procédé selon la revendication 4, caractérisé en ce que le traitement thermique améliorant les propriétés de basse émissivité consiste en un maintien à température élevée d'au moins 300°C, sous atmosphère réductrice, pendant une durée variant de quelques secondes à plusieurs heures, puis en un refroidissement sous atmosphère réductrice ou neutre jusqu'à une température où les propriétés de la couche ne sont plus altérées au contact d'une atmosphère oxydante.

6. Procédé selon la revendication 5, caractérisé en ce que le refroidissement sous atmosphère contrôlée réductrice ou neutre est pratiqué jusqu'à une température qui est au plus de 300°C.

7. Procédé selon l'une des revendications 5 et 6, caractérisé en ce que l'atmosphère réductrice ou neutre de traitement de la couche est constituée par $N_2 + X \% H_2$ où X est supérieur ou égal à 0 et notamment de l'ordre de 10.

8. Procédé selon la revendication 4, caractérisé en ce que le traitement thermique consiste en un chauffage intense pendant une durée inférieure à la seconde, en atmosphère contrôlée.

9. Procédé selon la revendication 8, caractérisé en ce que pour obtenir le chauffage intense et bref de la couche à traiter en atmosphère contrôlée soit réductrice, soit oxydante, on soumet, le substrat revêtu de la couche à traiter à l'action d'au moins une flamme réductrice ou au contraire oxydante, ladite flamme étant disposée du côté du revêtement.

10. Procédé selon la revendication 9, caractérisé en ce qu'on fait défiler le substrat, portant la couche mince, dans la flamme d'au moins un brûleur alimenté en gaz dont les caractéristiques réductrices ou oxydantes sont contrôlées et adaptées à la nature réductrice ou oxydante souhaitée du traitement.

11. Procédé selon la revendication 8, caractérisé en ce que le chauffage intense et bref de la couche à traiter en atmosphère réductrice ou au contraire oxydante est obtenu à l'aide d'au moins une torche à plasma microonde alimentée en gaz réducteur ou au contraire oxydant suivant l'effet désiré.

12. Procédé selon l'une des revendications 8 à 11, caractérisé en ce que plusieurs rangées de moyens de chauffage sont disposées de façon notamment à permettre une vitesse élevée de défilement du substrat.

13. Procédé selon l'une des revendications 8 à 12, caractérisé en ce qu'on modifie localement et/ou momentanément les conditions d'exposition de la couche au(x) brûleur(s) ou moyen(s) équivalent(s) de façon à obtenir une couche possédant des zones à propriétés différentes.

14. Procédé selon l'une des revendications 4 à 13, caractérisé en ce que le traitement thermique est pratiqué sur la ligne de fabrication du verre immédiatement après la formation de la couche.

15. Procédé selon l'une des revendications 4 à 13, caractérisé en ce que le traitement thermique est pratiqué sur une installation séparée de la ligne de fabrication du verre, notamment arche de recuisson annexe, ligne de trempe, ligne de bombage.

16. Procédé selon l'une des revendications 8 à 13, caractérisé en ce que le substrat en verre est trempé ou feuilleté avant traitement.

17. Vitrage obtenu par le procédé conforme à l'une des revendications 4 à 16, comprenant une couche d'oxydes d'indium et d'étain ayant une résistivité inférieure à $3.10^{-4}.\Omega.cm$ et une émissivité inférieure à 0,15.

18. Application du procédé selon l'une des revendications 8 à 17 à la fabrication de vitrages chauffants à couches avec des zones ayant des caractéristiques électriques différentes les unes des autres.

19. Application du procédé selon les revendications 8 à 17 à la fabrication de vitrages trempés et revêtus d'une couche mince à base d'oxyde d'indium, ayant des caractéristiques d'émissivité et de résistivité de bonne qualité, c'est-à-dire inférieure à 0,15 pour l'émissivité et inférieure à $3.10^{-4}$ ohm.cm pour la résistivité, le niveau de trempe étant tel qu'il autorise l'utilisation dudit vitrage en tant que vitrage de sécurité pour automobile.

20. Application du procédé selon les revendications 8 à 17 à la fabrication de vitrages feuilletés revêtus d'une couche mince à base d'oxyde d'indium ayant des caractéristiques d'émissivité et de résistivité modifiées par rapport à celles de la couche initiale.

**21.** Application du procédé selon les revendications 1 à 17 à la fabrication de substrats, en particulier en verre, revêtus d'une couche miroir métallique.

**22.** Application du procédé selon les revendications 1 à 17 à la recristallisation de couches minces initialement amorphes ou mal cristallisées.


**Patentansprüche**

**1.** Verfahren zur Herstellung einer dünnen transparenten und elektrisch leitenden Schicht aus Metalloxid(en) auf einem Substrat, insbesondere auf Glas, das im Aufsprühen von Metallverbindungen als Pulver in Suspension in einem Trägergas auf das auf erhöhte Temperatur gebrachte Substrat besteht, welche sich in Kontakt mit dem heißen Substrat zersetzen und unter Bildung der metallischen Oxidschicht oxidieren, dadurch gekennzeichnet, daß man auf das Substrat aus Indiumformiat oder aus einer Mischung aus Indiumformiat und einer oder mehreren Metallverbindungen mit einer Zersetzungstemperatur in der gleichen Größenordnung, wie der von Indiumformiat, gebildetes Pulver aufsprüht, wobei sich das Pulver in Kontakt mit dem Substrat unter Bildung einer dünnen Schicht auf Basis von Indiumoxid pyrolisiert.

**2.** Verfahren nach Anspruch 1, dadurch gekennzeichnet, daß mam auf das Substrat eine Pulvermischung aufsprüht, die Indiumformiat und Dibutylzinnoxid und/oder Dibutylzinndifluorid enthält.

**3.** Verfahren nach Anspruch 1, dadurch gekennzeichnet, daß man auf das Substrat Indiumformiatpulver und eine in das Trägergas, in dem sich das Pulver in Suspension befindet, eingemischte gasförmige Zinnverbindung, ausgewählt aus der von $SnCl_4$ und $BuSnCl_3$ gebildeten Gruppe, aufsprüht.

**4.** Verfahren nach einem der Ansprüche 1 bis 3, dadurch gekennzeichnet, daß die erhaltene dünne Schicht einer thermischen Behandlung in reduzierender oder oxidierender Atmosphäre, je nachdem, ob man die Schicht reduzieren oder oxidieren will, unterworfen wird, um die Zahl der Sauerstofflücken zu erhöhen und die Emissivität und den Widerstand der Schicht zu vermindern und dadurch ihre Eigenschaften bezüglich niedriger Emissivität und niedrigem Widerstand zu verbessern, oder andernfalls die Zahl der Sauerstofflücken zu vermindern, um die Emissivität und den Widerstand der Schicht zu erhöhen und dadurch die Eigenschaften bezüglich niedriger Emissivität, die sie zuvor besessen hat, zu verschlechtern.

**5.** Verfahren nach Anspruch 4, dadurch gekennzeichnet, daß die thermische Behandlung, die die Eigenschaft niedriger Emissivität verbessert, in der Beibehaltung einer erhöhten Temperatur von wenigstens 300°C unter reduzierender Atmosphäre während eines Zeitraums von wenigen Sekunden bis mehreren Stunden, und danach in der Abkühlung unter reduzierender oder neutraler Atmosphäre bis auf eine Temperatur, bei der sich die Eigenschaften der Schicht in Kontakt mit oxidierender Atmosphäre nicht mehr verändern, besteht.

**6.** Verfahren nach Anspruch 5, dadurch gekennzeichnet, daß die Abkühlung unter kontrollierter reduzierender oder neutraler Atmosphäre stattfindet und bis auf eine Temperatur erfolgt, die höchstens 300°C ist.

**7.** Verfahren nach einem der Ansprüche 5 und 6, dadurch gekennzeichnet, daß die reduzierende oder neutrale Atmosphäre zur Behandlung der Schicht aus $N_2 + X \% H_2$ gebildet wird, wobei X größer oder gleich 0 ist und insbesondere in der Größenordnung von 10 liegt.

**8.** Verfahren nach Anspruch 4, dadurch gekennzeichnet, daß die thermische Behandlung im intensiven Erhitzen über eine Zeitdauer von weniger als einer Sekunde in kontrollierter Atmosphäre stattfindet.

**9.** Verfahren nach Anspruch 8, dadurch gekennzeichnet, daß man zur intensiven und kurzen Erhitzung der in kontrollierter Atmosphäre, sei sie reduzierend oder oxidierend, zu behandelnden Schicht das mit der zu behandelnden Schicht überzogene Substrat der Einwirkung wenigstens einer reduzierenden oder andernfalls oxidierenden Flamme unterwirft, wobei die Flamme seitlich zur Beschichtung angeordnet ist.

**10.** Verfahren nach Anspruch 9, dadurch gekennzeichnet, daß man das Substrat, das die dünne Schicht aufweist, an der Flamme wenigstens eines Erhitzers vorbeiführt, der mit Gas versorgt wird, dessen reduzierende oder oxidierende Eigenschaften kontrolliert und an die gewünschte reduzierenden oder oxidierende Natur der Behandlung angepaßt werden.

11. Verfahren nach Anspruch 8, dadurch gekennzeichnet, daß das intensive und kurze Erhitzen der in reduzierender oder andernfalls in oxidierender Atmosphäre zu behandelnden Schicht mit Hilfe wenigstens einer Mikrowellen-Plasmafackel erzielt wird, die mit einem reduzierenden oder andernfalls oxidierenden Gas, je nach erwünschtem Effekt, versorgt wird.

12. Verfahren nach einem der Ansprüche 8 bis 11, dadurch gekennzeichnet, daß mehrere Bereiche mit Heizeinrichtungen auf eine Weise angeordnet sind, die insbesondere eine erhöhte Vorbeiführungsgeschwindigkeit des Substrats erlaubt.

13. Verfahren nach einem der Ansprüche 8 bis 12, dadurch gekennzeichnet, daß man die Einwirkungsbedingungen der Heizeinrichtung(en) oder äquivalenter Mittel auf eine Weise örtlich und/oder zeitlich modifiziert, um eine Schicht zu erzielen, die Zonen mit verschiedenen Eigenschaften besitzt.

14. Verfahren nach einem der Ansprüche 4 bis 13, dadurch gekennzeichnet, daß die thermische Behandlung auf der Fabrikationsstraße des Glases unmittelbar nach der Bildung der Schicht stattfindet.

15. Verfahren nach einem der Ansprüche 4 bis 13, dadurch gekennzeichnet, daß die thermische Behandlung auf einer von der Fabrikationsstraße des Glases getrennten Installation durchgeführt wird, insbesondere in einem angeschlossenen Nachglühofen, auf einer Vorspannstraße oder einer Bombierstraße.

16. Verfahren nach einem der Ansprüche 8 bis 13, dadurch gekennzeichnet, daß das Glassubstrat vor der Behandlung vorgespannt oder zu Schichten verbunden wird.

17. Nach dem Verfahren eines der Ansprüche 4 bis 16 erhaltene Verglasung, welche eine Schicht aus Indium- und Zinnoxid mit einem Widerstand von weniger als $3.10^{-4}$ .$\Omega$.cm und eine Emissivität von weniger als 0,15 aufweist.

18. Anwendung des Verfahrens nach einem der Ansprüche 8 bis 17 auf die Herstellung von Heizverglasungen, die Schichten mit Zonen aufweisen, die eine von der anderen verschiedene elektrische Eigenschaften haben.

19. Anwendung des Verfahrens nach Anspruch 8 bis 17 auf die Herstellung von vorgespanntem und mit einer dünnen Schicht auf Basis von Indiumoxid beschichteten Verglasungen mit guten Eigenschaften bezüglich der Emissivität und des Widerstands, d.h. von weniger als 0,15 bezüglich der Emissivität und weniger als $3.10^{-4}$ Ohm.cm bezüglich des Widerstands, wobei der Vorspannungsgrad so ist, daß er die Verwendung der Verglasung als Sicherheitsverglasung für Kraftfahrzeuge erlaubt.

20. Anwendung des Verfahrens nach Anspruch 8 bis 17 auf die Herstellung von mit einer dünnen Schicht auf Basis von Indiumoxid beschichtetem Verbundglas mit Eigenschaften bezüglich Emissivität und Widerstand, die gegenüber denen der ursprünglichen Schicht verändert sind.

21. Anwendung des Verfahrens nach Anspruch 1 bis 17 auf die Herstellung von Substraten, insbesondere aus Glas, die mit einer metallischen Spiegelschicht beschichtet sind.

22. Anwendung des Verfahrens nach Anspruch 1 bis 17 auf die Rekristallisation von ursprünglich amorphen oder schlecht kristallisierten dünnen Schichten.

## Claims

1. Process for forming a thin, transparent electrically conducting film of metallic oxide or oxides on a substrate, notably glass, the process consisting of projecting, onto said substrate raised to high temperature, metallic compounds in powder form in suspension in a carrier gas, which, on contact with the hot substrate, decompose and oxidize to form said film of metallic oxide or oxides, characterized in that there is projected, onto the substrate, powder formed by indium formate or by a mixture of indium formate and one or more metallic compounds having a decomposition temperature of the same order of magnitude as that of the indium formate, the powder pyrolizing on contact with the substrate to form a thin film based upon indium oxide.

2. Process according to Claim 1, characterized in that there is projected, onto the substrate, a powder of a mixture comprising indium formate and dibutyl tin oxide and/or dibutyl tin difluoride.

3. Process according to Claim 1, characterized in that there is projected, onto the substrate, powder of indium formate and a gaseous compound of tin chosen from the group formed of $SnCl_4$ and $BuSnCl_3$, mixed with the carrier gas in which said powder is in suspension.

4. Process according to one of Claims 1 to 3, characterized in that the thin film obtained is subjected to a thermal treatment in a reducing or oxidizing atmosphere depending upon whether it is desired to reduce or to oxidize the film, to increase the number of oxygen vacancies for the purpose of reducing the emissivity and the resistivity of the film and therefore improving its properties of low emissivity and low resistivity or, on the contrary, to reduce the number of oxygen vacancies for the purpose of raising the emissivity and the resistivity of the film and thus reducing the properties of low emissivity which it previously possessed.

5. Process according to Claim 4, characterized in that the thermal treatment improving the properties of low emissivity consists of holding at a high temperature of at least 300°C, under a reducing atmosphere, for a period varying from a few seconds to several hours, then of cooling under a reducing or neutral atmosphere to a temperature at which the properties of the film are no longer altered upon contact with an oxidizing atmosphere.

6. Process according to Claim 5, characterized in that the cooling under a controlled reducing or neutral atmosphere is carried out down to a temperature which is at most 300°C.

7. Process according to one of Claims 5 and 6, characterized in that the reducing or neutral atmosphere for treating the film is constituted of $N_2$ + X % $H_2$, where X is greater than or equal to 0 and notably of the order of 10.

8. Process according to Claim 4, characterized in that the thermal treatment consists of an intense heating for a duration of less than one second in a controlled atmosphere.

9. Process according to Claim 8, characterized in that, for achieving the intense, brief heating of the film to be treated in a controlled, either reducing or oxidizing, atmosphere, the substrate coated with the film to be treated is subjected to the action of at least one reducing or oxidizing flame, this flame being disposed on the coating side.

10. Process according to Claim 9, characterized in that the substrate, carrying the thin film, is caused to move through the flame of at least one burner supplied with gas, the reducing or oxidizing characteristics of which are controlled and are adapted to the reducing or oxidizing nature desired for the treatment.

11. Process according to Claim 8, characterized in that the intense, brief heating of the film to be treated in a reducing or oxidizing atmosphere is achieved by means of at least one microwave plasma torch, supplied with a reducing or oxidizing gas depending upon the effect desired.

12. Process according to one of Claims 8 to 11, characterized in that a plurality of rows of heating means are arranged in such a manner, notably, as to make possible a high speed of passage of the substrate.

13. Process according to one of Claims 8 to 12, characterized in that the conditions of exposure of the film to the burner or burners or equivalent means are modified locally and/or temporarily in such a manner as to obtain a film possessing zones of different properties.

14. Process according to one of Claims 4 to 13, characterized in that the thermal treatment is carried out on the production line for the glass immediately after the formation of the film.

15. Process according to one of Claims 4 to 13, characterized in that the thermal treatment is carried out on an installation separate from the production line for the glass, notably an annexed annealing furnace, a toughening line or a sheet bending line.

16. Process according to one of Claims 8 to 13, characterized in that the glass substrate is toughened or laminated before treatment.

17. Pane obtained by the process according to one of Claims 4 to 16, comprising a film of indium and tin oxides having a resistivity less than $3 \times 10^{-4}.\Omega.cm$ and an emissivity less than 0.15.

18. Application of the process according to one of Claims 8 to 17 to the production of heating panes having films with zones possessing different electrical characteristics from one to another.

19. Application of the process according to Claims 8 to 17 to the production of toughened panes coated with a thin film based upon indium oxide, having characteristics of emissivity and resistivity of good quality, that is to say less than 0.15 for the emissivity and less than $3 \times 10^{-4}$ ohm.cm for the resistivity, the level of toughening being such that it allows the use of said pane as a safety pane for automobiles.

20. Application of the process according to Claims 8 to 17 to the production of laminated panes coated with a thin film based upon indium oxide having characteristics of emissivity and resistivity modified by comparison with those of the initial film.

21. Application of the process according to Claims 1 to 17 to the production of substrates, in particular of glass, coated with a metallic mirror film.

22. Application of the process according to Claims 1 to 17 to the recrystallization of thin films, which initially were amorphous or badly crystallized.